Europäisches Patentamt

European Patent Office

Office européen des brevets

(19)

(11) Veröffentlichungsnummer: **0 141 773**
A2

(12)

## EUROPÄISCHE PATENTANMELDUNG

(21) Anmeldenummer: 84810402.2

(22) Anmeldetag: 14.08.84

(51) Int. Cl.⁴: **G 01 N 27/22**, G 01 N 33/28

(30) Priorität: 25.08.83  CH 4623/83

(43) Veröffentlichungstag der Anmeldung: 15.05.85
**Patentblatt 85/20**

(84) Benannte Vertragsstaaten: **AT BE DE FR GB IT LU NL SE**

(71) Anmelder: **Syntec Instruments AG,
Dättlikonerstrasse 5 Postfach 8, CH-8422 Pfungen (CH)**

(72) Erfinder: **Voney, Franz, Klotenerstrasse 8,
CH-8305 Dietlikon/Schweiz (CH)**

(54) **Bestimmung des Wassergehaltes in hydraulischen Flüssigkeiten.**

(57) Mittels zweier planparallelen Platten, die in die zu messende Flüssigkeit getaucht werden, wird die Kapazitätsänderung der Flüssigkeit infolge Wassergehaltszunahme mittels variablen Pulsweiten gemessen.

EP 0 141 773 A2

Die Erfindung bezieht sich auf ein Messgerät zur Bestimmung des Wassergehaltes in Flüssigkeiten mit niederen Dielektrizitätskonstanten, aus der mit Wasserzunahme verbundenen Aenderung der Kapazität der Flüssigkeit, mittels einer kapazitiven Messsonde, die zwei Elektroden besitzt, welche über einen Pulsweitenmodulator mit einer hochfrequenten Wechselspannungsquelle einerseits und einer Auswertelektronik andererseits verbunden sind.

Ein derartiges Messgerät wird in der englischen Patentschrift Nr. 1 578 527 für fliessendes Oel beschrieben. Die Elektroden dieses bekanntgewordenen Messgerätes bilden einen Messkondensator, der, alternativ zu einem Referenzkondensator, in einem Oszillatorkreis, eine frequenzbestimmende Funktion ausübt. Es hat den Nachteil, dass relativ geringe Mengen von Wasser bei den vorgeschlagenen Frequenzen nur schwierig zu erfassen sind.

In der französischen Patentanmeldung Nr. 74 17295 wird ein weiteres Gerät zur Bestimmung des Wassergehaltes in Hydraulik-Oelen beschrieben. Die Mehrfachelektroden dieses Gerätes werden in die Flüssigkeit eingetaucht und bilden zusammen mit einer Spule und einer Anregungsquelle einen Schwingkreis, dessen Resonanzfrequenz von der Dielektrizitätskonstanten der zu untersuchenden Flüssigkeit abhängt. Mit dieser Methode lassen sich auch geringe Kapazitätsänderungen feststellen. Der Nachteil dieses Gerätes beruht jedoch darin, dass immer eine Vergleichs-

FLÜSSIGKEIT VORHANDEN SEIN MUSS UM DAS GERÄT VORHER ZU EICHEN. EIN WEITERER NACHTEIL LIEGT IN DER ANORDNUNG DER MEHRFACHELEKTRODEN, DA DIESE NACH DER BENUTZUNG UMSTÄNDLICH GEREINIGT WERDEN MÜSSEN, ANSONSTEN DAS NÄCHSTE MESSRESULTAT VERFÄLSCHT WÜRDE.

DER ERFINDUNG LIEGT DIE AUFGABE ZUGRUNDE, EIN LEICHT ZU HANDHABENDES, TRAGBARES, BATTERIEGESPIESENES GERÄT ZUR SIEDEPUNKTBESTIMMUNG VON HYDRAULIK-OELEN ZU SCHAFFEN. SOLCHE GERÄTE EXISTIEREN ZWAR, DOCH SIND SIE ÜN- HANDLICH IN DER ANWENDUNG ODER NICHT BEFRIEDIGEND IN DER AUSSAGEKRAFT. DA HYDRAULISCHE FLÜSSIGKEITEN HYGROSKOPISCH SIND, LIEGT ES NAHE, DEN ZUSAMMENHANG ZWISCHEN WASSERGEHALT UND SIEDEPUNKT AUSZUNUTZEN. DIESE AUFGABE WIRD GEMÄSS DER ERFINDUNG DADURCH GELÖST, DASS EINE SONDE IN DIE ZU UNTERSUCHENDE FLÜSSIGKEIT GETAUCHT WIRD, UM DEREN KAPAZITÄTSÄNDERUNG ZU MESSEN.

UM DIE ERFINDUNG BESSER ZU VERSTEHEN, WIRD IM FOLGENDEN ANHAND DER FIGUREN, IN DENEN DIE VERSCHIEDENEN TEILE DURCH NUMMERN GEKENNZEICHNET SIND, EINE MÖGLICHE ANWENDUNG ERLÄUTERT.

FIG. 1 : BLOCKSCHEMA DES GERÄTES

FIG. 2 : SONDE

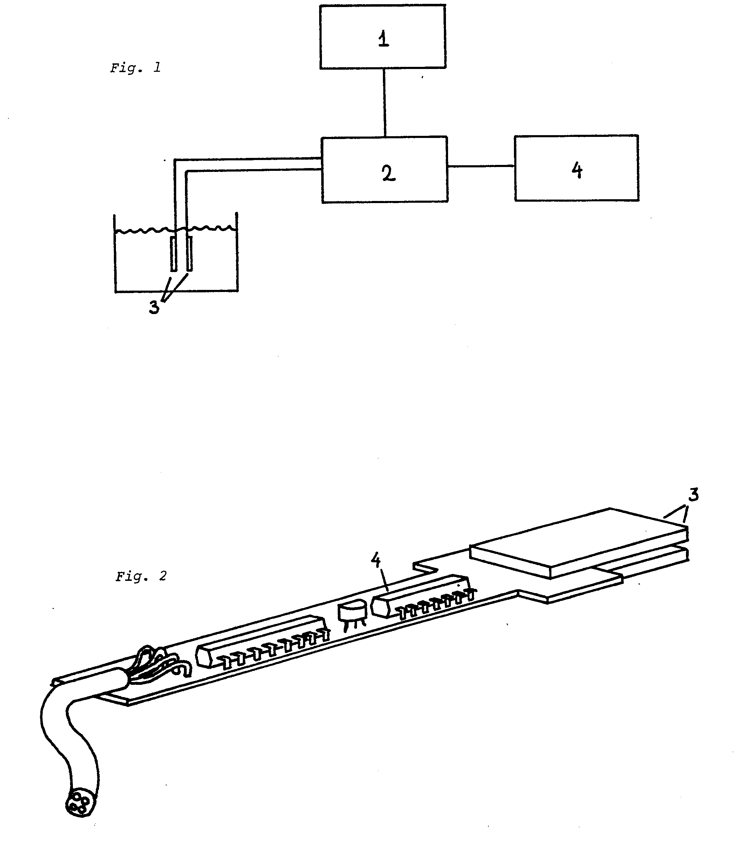

DURCH DIE VERWENDUNG DER IN FIG. 2 DARGESTELLTEN SONDE UND DER IN FIG. 1 DARGESTELLTEN ANORDNUNG KÖNNEN SEHR KLEINE AENDERUNGEN DER KAPAZITÄT DER ZU UNTERSUCHENDEN FLÜSSIGKEIT IN AUSSAGEKRÄFTIGE DATEN ÜBERSETZT WERDEN.

DER QUARZGESTEUERTE OSZILLATOR 1 REGT DEN PULSWEITENMODULATOR 2 AN, DESSEN PULSWEITEN VON DER KAPAZITÄT
DER SONDE 3 ABHÄNGT. DIE PULSWEITEN WERDEN VON DER AUSWERTELEKTRONIK 4 AUSGEWERTET UND ENTWEDER ALS ANALOGE
SPANNUNG ODER ALS DISKRETE WERTE EINES LEUCHTBALKENS MIT
WARNGRENZEN AUSGEGEBEN.

## Bestimmung des Wassergehaltes in hydraulischen Flüssigkeiten.

Patentanspruch :

1.

Methode zur Bestimmung des Wassergehaltes in schlecht leitenden Flüssigkeiten mit niederen Dielektrizitätskonstanten, aus der Zunahme der Dielektrizitätskonstanten mit zunehmendem Wassergehalt, mittels einer kapazitiven Mess – Sonde, die zwei Elektroden besitzt, welche über einen Pulsweitenmodulator mit einer hochfrequenten Wechselspannungsquelle einerseits und einer Auswertelektronik andererseits verbunden sind, dadurch gekennzeichnet, dass die Elektroden, zwei planparallele Platten, in die Flüssigkeit eingetaucht werden und die Kapazitätsänderung mittels variabler Pulsweiten gemessen wird.

Fig. 1

1

2

4

3

Fig. 2

4

3